# EUROPEAN PATENT SPECIFICATION

(11) **EP 1 968 996 B1**
(45) Date of publication and mention of the grant of the patent: **30.09.2009**
(21) Application number: 06819816.7
(22) Date of filing: 28.11.2006
(51) Int. Cl.: C07K 1/107

(54) **PROCESS FOR THE PREPARATION OF FERRI-SUCCINYLCASEIN**
VERFAHREN ZUR HERSTELLUNG VON FERRISUCCINYLCASEIN
PROCÉDÉ DE SYNTHÈSE DE FERRI-SUCCINYLCASÉINE

(30) Priority: 06.12.2005 EP 05111733
(43) Date of publication of application: 17.09.2008
(73) Proprietor: VIFOR (INTERNATIONAL) AG, 9001 St. Gallen (CH)
(72) Inventor: KLOTZ, Jürgen, Dr., CH-9053 Teufen (CH); REIM, Stefan, CH-9014 St. Gallen (CH); PHILIPP, Erik, Dr., CH-9303 Wittenbach (CH); MÜLLER, Hans-Martin, CH-9032 Engelburg (CH); GEISSER, Peter, Dr., CH-9014 St. Gallen (CH)
(74) Representative: Gille Hrabal Struck Neidlein Prop Roos
(86) International application number: PCT/EP2006/068994
(87) International publication number: WO 2007/065812

(56) References cited:
- EP-A- 0 939 083
- WO-A-20/06021843
- GB-A- 2 115 821
- CREMONESI P ET AL: "IRON DERIVATIVES OF MODIFIED MILK PROTEIN" ARZNEIMITTEL FORSCHUNG. DRUG RESEARCH, ECV EDITIO CANTOR VERLAG, AULENDORF, DE, vol. 34, no. 9, 1984, pages 948-952, XP001207730 ISSN: 0004-4172
- PAGELLA P G ET AL: "Pharmacological and Toxicological Studies on an Iron Succinyl-Protein Complex (ITF282) for Oral Treatment of Iron Deficiency Anemia" ARZNEIMITTEL FORSCHUNG. DRUG RESEARCH, ECV EDITIO CANTOR VERLAG, AULENDORF, DE, vol. 34 (II), no. 9, 1984, pages 952-958, XP002172941 ISSN: 0004-4172

## Description

The present invention relates to a new process for the preparation of ferri-succinylcasein.

GB-A-2115821 discloses the preparation of iron-containing succinylated proteins. The preparation includes the reaction of milk powder, milk proteins, egg proteins, bovine serum proteins, pig liver proteins or soya proteins with succinic anhydride under alkaline conditions, centrifugation or filtration of the opalesecent solution and precipitation upon acidification. The precipitate is again separated by centrifugation or filtration and re-suspended in water. Thereafter the separated precipitate is dissolved again under weak alkaline conditions, the solution is centrifugated or filtrated and again acidified to form a precipitate of succinylated proteins. For the reaction with the iron salt the precipitate is again dissolved under weak alkaline conditions. Despite the laborious purification steps of precipitation and re-precipitation of the succinylated proteins to purify the succinylated proteins the process described in GB-A-2115821 suffers from the disadvantage in that, in particular, insoluble derivatives in the form of a mucilage are formed, which is difficult to eliminate (EP-A2-0939083). In an attempt to overcome these disadvantages of GB-A-2115821 the European patent application EP-A2-0 939 083 describes a ferric complex of succinylated casein which is obtained from food-grade casein and which is used in the treatment of pathological conditions linked to iron deficiency. The ferric-succinylcasein is prepared via an complicated synthesis involving the reaction of casein with succinic anhydride to form succinylcasein which is subsequently reacted with ferric chloride. After the precipitation of the succinylcasein in the first step, the succinylcasein obtained is dissolved again under laborious dilaceration. Dilaceration is a process involving high mechanical energy characterized by the use of pumps which enables elimination of aggregations of suspended solid material which cannot be eliminated by mere mechanical agitation. Dilaceration processes are carried out by using special dilaceration pumps in which the movement of a propeller is associated to the movement of a grinding gear. The aqueous solution of succinylcasein obtained after dilaceration is then reacted with ferric chloride. Also the ferri-succinylcasein obtained is again dissolved under laborious dilacerations. Accordingly the process according to EP-A2-0 939 083 is disadvantageous as these separation and dilaceration steps make the process complicated, time-, cost- and energy-consuming. Moreover, comparatively high amounts of succinic anhydride have to be used in the process to result in an adequate yield of the desired product. Also the process of GB-A-21 15821 is quite disadvantageous, in particular, on an industrial scale as it requires the steps of precipitation, re-dissolving, re-precipitation and re-dissolving of the succinylated proteins before the reaction with the iron salt.

EP-A1-0319664 also discloses a process for the preparation of iron complexes with succinylated polypeptides, which have been subjected to enzymatic degradation. With respect to the preparation of the succinylated polypeptides used therein, as a starting material such document refers in turn to IT 1150213, corresponding to the GB-A-21 15821 mentioned before.

WO 2006/021843, published after the priority date of the present application discloses a process for the preparation of iron succinyl casein. Similar to EP-A2-0 939 083 and GB-A-2115821 also this document describes in the examples the manufacture of succinyl casein by reacting casein with succinyl anhydride in alkaline solution. Subsequently the succinyl casein is precipitated by addition of HCl, The precipitated succinyl casein is then again completely dissolved and filtrated and then subjected to the reaction with iron chloride. That is, also the process of WO 2006/021843 discloses the additional steps of isolation by filtration, washing, damp granulation, dissolving, filtration, of the succinyl casein intermediate before a solution - rather than a suspension - of the succinyl casein intermediate is subjected to the reaction with iron chloride. In addition WO 2006/021843 is characterized by an additional granulation step for the precipitated succinyl casein intermediate before the redissolution step, wherein the solution of the succinyl casein is obtained, to be reacted with the iron chloride.

Accordingly the relevant prior art obviously has considered that additional re-dissolving and re-precipitation steps for the succinyl casein intermediate are essential in the preparation of iron succinyl casein, and further that it is essential to react a solution rather than a suspension of the succinyl casein intermediate with the iron chloride.

In a patent search carried out after the priority date of this application by the Austrian Patent Office the following additional prior art documents have been identified, which, however, turned out to be of less or no importance: AU 652021 B, EP 0739634 B1 , EP 0243322 B1, US 6994876 B1, GB 1475577 A, US 2006/147552, WO 2006/001429 und WO 2006/001430.

As a consequence, it has been an object of the present application to provide a new process for the preparation of ferri-succinylcasein which is characterized by,
- a one pot reaction,
- no intermediate isolation-, purification- and/or dilacerations steps,
- shorter processing times,
- increased concentrations leading to higher volume efficiencies,
- less amounts of succinic anhydride,
- increased material resistance because of less aggressive pH-values.

The present application relates to a less complicated, and more time-, cost- and energy efficient process which is leading to the desired product quality in a high yield. Thereby, the ferri-succinylcasein complex obtained should allow the administration of precise and reproducible quantities of iron mainly without including side effects. The present inventors could show that ferri-succinylcasein can be prepared in a much more easy process, which allows to avoid any intermediate isolation step, purification step, in particular, re-precipitation step, or dilaceration process step for the succinylated protein intermediate.

Accordingly the present invention provides a new process for the preparation of ferri-succinylcasein comprising the following steps:
(a) reacting casein with at least one succinylation agent, preferably succinic anhydride, to form an aqueous suspension of succinylcasein, and
(b) reacting the aqueous suspension of succinylcasein obtained in step (a) with at least one iron salt to form ferri-succinylcasein.

Accordingly such process differs from the process described in GB-A-21 15821 in that not a solution of the succinylated protein, which has been obtained by precipitation, dissolving, re-precipitation and re-dissolving is reacted with the iron salt, but an aqueous suspension, obtained by reacting casein with succinic anhydride and subsequent acidification, is directly, that is, without delaceration and/or purification, that is, re-precipitation step, reacted with the iron salt.

Similarly the process of the invention differs from the process disclosed in EP 0939083 A2, in particular, again in that not a solution of the succinylcasein is reacted with the iron salt but the suspension obtained by the acid precipitation in the step (a) of preparing the succinylcasein is directly reacted with the iron salt. Thereby the time-, energy-, and thus cost-consuming dilaceration steps to bring the succinylcasein obtained in step (a) into solution surprisingly can be completely omitted without any disadvantages. Aqueous suspension in contrast to an aqueous solution means that a suspension contains insolubles, which can be separated by filtration.

So - in contrast to the present invention - in both prior art documents (EP-A2-0939083 and GB-A-2115821) a solution of the succinylated protein rather than a suspension is contacted with the iron salt. In addition both prior art processes require the additional purification steps for the succinylated protein of dilaceration (EP-A2-0939083) and re-precipitation (GB-A-2115821), which are not carried out in the process of the present invention. Accordingly the process of the present invention provides a significant advantage over the prior art, in particular, on the industrial scale.

That is, the aqueous suspension of the succinylated casein obtained in step (a) upon reacting the succinylating agent with casein is (directly) subjected to the reaction with the iron salt. In particular, no separation and/or isolation steps of the succinylated casein, and no purification steps, like re-precipitation or delaceration steps are performed between the reaction of the succinylating agent with casein in step (a) and the reaction of the succinylated casein and the iron salt in step (b).

In a preferred embodiment of the invention step (a) comprises any of the sub-steps:
(a 1) suspending casein in water, preferably at higher concentrations (in contrast to EP-A2-0939083 and GB-A-21 1 5821)
(a2) if necessary, adjusting the pH value of the aqueous suspension to at least 6,
(a3) adding at least one succinylation agent, preferably succinic anhydride, while maintaining a pH value of at least 6 by the addition of at least one base.

In step (a1) preferably the weight ratio of casein to water is adjusted in the range of 1 : 1 to 1 : 100, preferably 1 : 2 to 1 : 15, more preferably 1 : 4 to 1 : 8 (m/m). It has been surprisingly ascertained that, in the process of the invention the initial amount of water can be reduced to a minimum and thus the volume efficiency of the process can be significantly increased.

On stirring the casein in water usually a pH of about 5 appears. Accordingly the process usually comprises the adjustment of a pH value of the aqueous suspension of casein in water to at least 6, preferably at least 7 (step (a2)), which is usually achieved by the addition of one or more bases, which include - without being limited to these: alkali or alkaline earth metal salt, like hydroxides, oxides and/or carbonates. Preferred are alkali metal hydroxides, like sodium or potassium hydroxide, in particular, sodium hydroxide. In step (a2) the pH value is still more preferably adjusted to at least 7.5, and still more preferably to about 8. After having prepared the aqueous suspension of the casein of a desired pH value, the succinylation agent, preferably succinic anhydride is added in step (a3), while maintaining a pH value of at least 7 by the addition of at least one base. The base is preferably the same as the one used in step (a2). Succinic anhydride is usually added in powder form. The succinic anhydride can be added to the casein continuously or discontinuously, for example in one or more than one temporally separated portions. Temporally separated means that the time period between two additions is preferably at least 30 seconds, more preferably at least 60 seconds and still more preferably at least 300 seconds. It has been surprisingly ascertained that, in the process of the invention the amount of required succinic anhydride can be reduced. Thus, in step (a) of the process according to the present invention the weight ratio of casein to succinic anhydride is preferably at least 3.5 to 1 , more preferably at least 4 : 1 , more preferably at least 5 : 1 . In a preferred embodiment of the present invention, the weight-ratio of the casein to succinic anhydride is at most 1 2 : 1 , preferably at most 1 0 : 1 , still more preferably at most about 8 : 1 . Since it is usually aspired to succinylate practically all the available amino residues in the casein (i.e., degree of substitution of more than 90 %; based on the determination using the ninhydrin method (Yemm, Cocking; Succinylation reaction, Analyst 80, 209)) corresponding to a content of succinic acid of about 7.5 % (m/m) as determined by gas chromatography (Cremonesi, P. & Caramazza. I (1993), International Journal of Clinical Pharmacology, Therapy and Toxicology, 31:40-51) the minimum weight ratio of succinic acid anhydride to casein is about 1 : 1 2.

According to the present invention, it is preferred to use food-grade casein, i.e. casein used for food purposes. By the term "food grade casein" is meant casein obtained from milk coming from strictly controlled breeding farms. These products present a level of microbiological purity with less than 10³ UFC/g for bacteria and with less than 10² UFC/g for moulds. The use of food-grade casein leads to particularly pure products as compared to similar complexes obtained starting from usual milk proteins.

Furthermore, it is preferred that the succinic anhydride is added to the casein during a time period of at least 10 minutes, more preferably of at least 15 minutes, more preferably of at least 20 minutes.

The reaction time after succinic anhydride addition is generally less than 60 minutes, preferably less than 45 minutes, more preferably less than 30 minutes.

The reaction of step (a) is usually performed at a temperature of about 10 to 40°C, preferably around room temperature (15 to 25°C).

After the reaction of succinic anhydride and casein, the resulting succinylated casein is in general precipitated by acidification to a pH of from 2 to 7, preferably of from 2.5 to 6.5, more preferably of from 3.0 to 4.5, by adding a suitable acid to the resulting solution (step (a4)). Specific examples of usable acids are aqueous solutions of hydrochloric acid and sulphuric acid, with hydrochloric acid being the most preferred acid.

Accordingly in step (a) there is usually obtained an acid suspension of succinylcasein having a pH value of preferably below 7, more preferably below 6.

In the process of the invention the above obtained normally acid aqueous suspension of the succinylcasein obtained in step (a) is usually used without further isolation, purification and dilacerations steps in the next step (b) of reaction with the iron salt. In particular, the precipitated succinylcasein obtained in step (a) in not brought again into solution, in particular, by laborious dilaceration processes, before reaction with the iron salt.

Thus in step (b) the acid aqueous suspension of succinylcasein obtained in step (a) having a pH value of preferably less than 7, more preferably less than 6 is directly reacted with at least one iron salt, that is, in particular, without further isolation, purification and/or delaceration steps. In contrast thereto in the processes of EP 0939083 A2 and GB-A-2115821 the iron salt is added to an alkaline solution of complicated purified succinylcasein which process is likely to cause the formation of considerable amounts of insoluble iron hydroxides precipitating at alkaline pH, which need again to be separated causing a loss of iron in the process.

In step (b) of the process according to the present invention, the precipitated succinylated casein is directly reacted with at least one iron source, preferably at least one iron salt, which is selected from the group consisting of ferric chloride, and other iron(III)-containing salts like sulphate, aspartate, fumarate, citrate, gluconate, glycinate, lactate and oxalate. Most preferred is iron(III)-chloride (ferric chloride).

Usually, the iron salt is used as an aqueous solution in step (b) of the process according to the present invention,

If ferric chloride is used as iron source in step (b), the reaction of the succinylcasein with the ferric chloride is preferably performed with a solution of ferric chloride with an iron content of from 0.5 to 20 % (m/m), more preferably of from 1 to 15 % (m/m), Higher concentrations of ferric chloride are preferred, because a shorter dosing time and a higher volume efficiency result. The dosing time for the solution of ferric chloride into the succinylcasein suspension is preferably from 1 to 40 min, more preferably from 2 to 20 min, most preferably from 3 to 15 min,

During the addition of the iron source in step (b) of the process according to the present invention, the pH is preferably maintained at a value of from 2 to 6, more preferably of from 2.5 to 5.0 and still more preferably of from 3 to 4 by adding an adequate amount of a base to the reaction medium to drive the iron complex formation. Regarding suitable bases it is referred to those mentioned above, with aqueous solutions of NaOH being preferred. Higher pH value are avoided to prevent the formation of iron hydroxides,

After the addition of the iron source to the succinylcasein suspension, the resulting mixture is preferably stirred at around room temperature (15 to 25°C).

Thereafter, preferably in step (c) the resulting ferri-succinylcasein obtained in an aqueous suspension is dissolved by the addiction of at least one base, to form an aqueous composition comprising dissolved ferri-succinylcasein. In step (c) the pH of the reaction mixture is in general adjusted to a value of at least 7.0, preferably from 7 to 11, more preferably from 7.5 to 9.5, by adding a suitable base. Regarding suitable bases it is referred to those mentioned above, with aqueous solutions of NaOH being preferred.

At the same time the suspension is preferably stirred at room temperature or slightly heated up to temperatures of preferably at most 50 °C, more preferably at most 45 °C, most preferably at most 40 °C, preferably for about 10 to 120 min, more preferably of from 20 to 100 min, most preferably of from 30 to 80 min.

Remaining insoluble matter is usually separated, preferably by filtration, from the aqueous composition comprising dissolved ferri-succinylcasein to obtain a normally alkaline aqueous solution of ferri-succinylcasein (step (d)).

In contrast to EP 0939083 A2 in the process of the present invention it is also not necessary to subject the ferri-succinylcasein suspension to a time and cost intensive isolation and/or dilaceration procedure to obtain the alkaline product solution. Thus, in a preferred embodiment of the process according to the present invention also an isolation and/or dilaceration step to obtain an alkaline solution of the ferri-succinylcasein is omitted.

Preferably in a further step (e) ferri-succinylcasein is precipitated from said aqueous solution by adding at least one acid, and recovering the ferri-succinylcasein obtained. Precipitation of ferri-succinylcasein in step (e) is preferably carried out at a pH of from 2 to 6, more preferably of from 3 to 5. In contrast to EP 0939083 A2 and GB 2115821 the pH range can be elevated to 3 or even more than 3 as it results in a less aggressive product suspension, which allows the application of stainless steel devices. Higher pH values are disadvantageous since the solubility of the ferri-succinylcasein at higher pH values might lead to a loss in yield.

Said acid to be used for the precipitation of the ferri-succinylcasein in step (e) is preferably diluted hydrochloric acid of a concentration of about 15 to 25 weight-%. Alternatively sulphuric, acetic, malonic, malic, citric, tartaric and lactic acid can be used.

According to the present invention, the crude product comprising ferri-succinylcasein is generally recovered by filtration or centrifugation, washing (e.g. with water) and drying, for example at a rotavap or vacuum dryer. In particular, the drying is performed under reduced pressure and higher temperatures than room temperature, e.g. at 50 to 100 °C, preferably at 60 to 90 °C, more preferably at 70 to 80 °C, for several hours, like 2 to 50 hours, preferably 10 to 40 hours preferably at a reduced pressure of e.g. 10 to 200 mbar, preferably 20 to 150 mbar. The residual water content is usually about less than 1 0 wt-%.

The resulting ferri-succinylcasein complex usually has an iron content of about 4 to 6, preferably about 5 wt-% based on iron(III).

In a preferred embodiment instead of drying the ferri-succinylcasein obtained it is also possible to process the wet material directly into a pharmaceutical formulation for oral administration, preferably into a liquid pharmaceutical formulation.

The process according to the present invention is preferably a one-pot process, and preferably comprises the following steps:
(a) Reacting casein, preferably at higher concentrations, i.e., at weight ratios of casein to water of 1 : 2 to 1 : 15, more preferably 1 : 4 to 1 : 8 with succinic anhydride to form an aqueous suspension of succinylcasein,
(b) Reacting said aqueous suspension of succinylcasein obtained in step (a) directly, that is, without intermediate isolation, purification and/or dilaceration steps with at least one iron salt to form ferri-succinylcasein, which is preferably substantially free from insoluble iron hydroxides,
(c) Dissolving said ferri-succinylcasein by the addition of at least one base, preferably without intermediate isolation, purification and/or dilaceration steps, to form an aqueous composition comprising dissolved ferri-succinylcasein,
(d) Separating insoluble matter from said aqueous composition comprising dissolved ferri-succinylcasein to obtain an aqueous solution of ferri-succinylcasein (this separation, in particular, by filtration is mainly carried out because of pharmaceutical requirements),
(e) Precipitating ferri-succinylcasein from said aqueous solution preferably at elevated pH values resulting in less aggressive product suspensions by adding at least one acid, and recovering the ferri-succinylcasein obtained, and
(f) Drying said ferri-succinylcasein obtained or further processing the wet ferri-succinylcasein obtained directly into a pharmaceutical formulation, preferably into a liquid pharmaceutical formulation.

In a preferred embodiment the process of the invention consists essentially of those product steps (a) to (f).

The process according to the invention can be carried out discontinuously (that is, batchwise) and/or partially continuously.

It represents one of the further advantages of the process according to the invention that, in view, of the great simplification achieved compared to the prior art, it can be performed at least partially continuously.

In a preferred embodiment, in particular, the work-up after step (c) mentioned above, that is, after dissolving the ferri-succinylcasein by the addition of at least one base, is performed continuously. More specifically, after step (c), the aqueous composition, comprising dissolved ferri-succinylcasein, is continuously discharged from the reactor and preferably diluted with water preferably in a static pipe mixer.

The optimum conditions of the dilution with water depend inter alia on the temperature of the aqueous composition, comprising dissolved ferri-succinylcasein, obtained in step (c). In case that the aqueous composition, obtained in step (c), has a temperature of more than 35° C, or more than 40°C, like for example 50°C, the water used for diluting has preferably a similar temperature as the aqueous composition, comprising dissolved ferri-succinylcasein, obtained in step (c). In particular, the temperature of the water used for diluting is preferably within the range of ± 5°C of the temperature of the aqueous composition, comprising dissolved ferri-succinylcasein, obtained in step (c). The volume ratio:
aqueous composition, comprising dissolved ferri-succinylcasein, obtained in step (c) / water used for diluting
is preferably in the range of about 1 : 1 to 1 : 10, more preferably 1 : 2 to 1 : 6, and most preferably 1 : 4.

In case that the aqueous composition, obtained in step (c), has a temperature of less than 35° C, like for example 30°C, and the water used for diluting has in particular a temperature within the range of 25 ± 5°C. the volume ratio:
aqueous composition, comprising dissolved ferri-succinylcasein, obtained in step (c) / water used for diluting
is preferably in the range of about 1 : 2 to 1 : 10, more preferably 1 : 4 to 1 : 8, and most preferably 1 ; 6.

After dilution of the aqueous composition, comprising dissolved ferri-succinylcasein, obtained in step (c), with water, the resulting diluted aqueous composition is preferably filtrated to remove any insolubles (corresponding to step (d)).

Thereafter, the preferably filtrated and diluted aqueous composition, comprising dissolved ferri-succinylcasein, is brought into contact with a liquid acid medium preferably in a further static pipe mixer to precipitate the ferri-succinylcasein (corresponding to step (e)). Said acid medium which is preferably diluted aqueous HCl (15 to 25 wt-%) is brought into contact with the diluted aqueous composition, comprising dissolved ferri-succinylcasein, in such a ratio that a pH of preferably 2 to 6, more preferably from 2.5 to 5, more preferably of from 3 to 4 is adjusted.

After precipitation the aqueous suspension comprising ferri-succinylcasein is generally recovered by filtration or centrifugationand washed (e.g. with water). On lab scale, these steps are preferably discontinuously, on production scale isolation and washing can be performed continuously using a decanter or a centrifugation device, respectively. Drying for example is performed at a rotavap or vacuum dryer as described above (step (f)). This step is preferably performed discontinuously on lab and production scale.

The advantages of a continous work-up as described above include:
- Higher batch sizes are possible (synthesis in a reactor of defined volume, work-up outside),
- work-up procedure is independent from batch sizes,
- improved consistence of precipitated product (less sticky).

Preferably according to the present invention the above mentioned steps (a) to (c) are performed discontinuously (i.e. batchwise) and the above mentioned steps (d) to (f) are performed continuously. According to the invention it is also possible to combine more than one batch-reactors wherein steps (a) to (c) are performed discontinuously with one continous work-up line (steps (d) to (f), and operating the batch-reactors in a swing mode, so that the continous work-up can be performed essentially without any interruption.

The process described in accordance with the present invention makes it possible to obtain ferri-succinylcasein free from residues or from iron derivatives that are insoluble or poorly soluble in water. In particular, the product is found to be completely soluble at neutral/alkaline pH values, i.e., the ones typical of the intestinal tract, thus guaranteeing iron bioavailability for the purpose of intestinal absorption.

The ferri-succinylcasein complexes according to the present invention have a constant composition, present excellent pharmacological activity linked to their behaviour as iron carriers and accordingly have a very low incidence of side effects. The therapeutic use of this product does not involve the disadvantages of known iron-based compounds, in particular gastric lesions. Furthermore, the product according to the present invention can be adequately compounded in pharmaceutical formulations, in particular, suitable for oral administration.

Thus the process of the invention preferably further comprises the step of manufacturing a pharmaceutical dosage from the ferri-succinylcasein obtained in the process above, wherein the dosage form is usually for oral administration.

Such process usually comprises formulating the ferri-succinylcasein with at least one suitable pharmaceutically acceptable adjuvant, diluent or carrier. Examples of adjuvants, diluents or carriers suitable for oral administration are for example microcyrstalline cellulose, calcium phosphate, diatomaceous earth, a sugar such as lactose, dextrose or mannitol, talc, stearic acid, starch, sodium bicarbonate and/or gelatine. The compositions may also contain suitable preserving, stabilising and wetting agents, solubilisers, sweetening and colouring agents and flavourings.

Suitable oral dosage forms include for example tablets, coated tablets, capsules, dragees, elixirs, lozenges, pellets, powders, solutions, suspensions, syrups, drinkable vials, juices. The pharmaceutical compositions may, if desired, be formulated in sustained release form.

The daily dosage depends of course on the particular subject to be treated and on the condition and disease to be treated. An average daily dosage is for example between 10 and 500 mg iron per day. For example patients with an iron-deficiency take 2 to 3-times daily about 100 mg iron, and pregnant woman take 1 to 2 times daily 60 mg iron.

The process of the invention preferably further comprises the use of the ferri-succinylcasein obtained for the manufacture of a medicament, which is preferably for the treatment of a patient suffering from any symptoms of an iron deficiency.

Such symptoms include for example: fatigue, lack of energy, poor concentration, reduced efficiency, difficulties to find the right words, forgetfulness, abnormal paleness or lack of color of the skin, irritability, increased heart rate (tachycardia), sore or swollen tongue, enlarged spleen, a desire to eat peculiar substances (pica), headaches, lack of appetite, increased susceptibility of infection, depressive dysphoria.

The present application further describes the use of the ferri-succinylcasein obtained for the manufacture of a medicament for the treatment of iron-deficiency anemia, in particular, iron-deficiency anemia in pregnancy, latent iron-deficiency anemia in children and adolescents, iron-deficiency anemia due to gastrointestinal tract abnormalities, iron-deficiency anemia due to blood loss, like for example gastrointestinal bleeding (e.g. due to ulcers, carcinomas, hemorrhoids, inflammatory disorders, ingestion of acetylsalicylic acid), menstrual bleeding, or injury, iron-deficiency anemia due to sprue, iron-deficiency anemia due to decreased dietary iron, iron-deficiency-related immunodeficiency, iron-deficiency-related impairment of the cerebral function, or Restless Leg Syndrome (RLS).

The following examples are reported to the purpose of illustrating without limitation of the invention,

### Examples:

### (If not otherwise indicated all percentages refer to m/m).

### Preparation of ferri-succinylcasein

### Example 1

120 g casein are suspended in 1620 g water at a temperature of 20°C. The pH value is adjusted to 8 by the addition of 9.8 ml 30 % NaOH solution within 40 min. 22 g succinic anhydride are added in four portions within 20 min. During that time the pH value is maintained at 8 by the addition of 35.3 ml 30 % NaOH solution. The resulting solution is stirred for 30 min, maintaining the pH value at 8. The succinylcasein is precipitated by adjustment of the pH value to 4 with 66.1 ml 20 % HCl solution. 57.2 g of a FeCl₃-solution (12 %) are added within 10 min, maintaining the pH value at 4 by the addition of 32.3 ml 30 % NaOH solution. The resulting suspension is stirred for 1 hour at a pH value of 4 and then heated up to 30°C within 20 min. The pH value is adjusted to 6 by the addition of 27.6 ml 30 % NaOH solution. 2.45 g propyl para-hydroxybenzoate and 9, 14 g methyl para-hydroxybenzoate dissolved in 30 ml water are added. The pH value is adjusted to 9 by the addition of 19.7 ml 30 % NaOH solution. The solution is stirred for 1 hour at 50°C and a pH value of 9. After filtration the product is precipitated by the addition of 82.2 ml 20 % HCl solution until a pH value of 3.6 is obtained. The product is filtered, washed with 1 I water and then dried for 5 hours at a temperature of 75°C and a pressure of 50 mbar on a rotavap. The product is further dried for 15 hours at 75 to 80 °C and a pressure of 125 mbar in a vacuum dryer. 127 g of ferri-succinylcasein are obtained.

### Example 2

120 g casein are suspended in 1622 g water at a temperature of 20 °C. The pH value is adjusted to 8 by the addition of 9.8 ml 30 % NaOH solution within 40 min. 20 g succinic anhydride are added in four portions within 20 min. During that time the pH value is maintained at 8 by the addition of 32.8 ml 30 % NaOH solution. The resulting solution is stirred for 30 min, maintaining the pH value at 8. The succinylcasein is precipitated by adjustment of the pH value to 4 with 60.5 ml 20 % HCl solutions. 57.2 g of a FeCl₃-solution (12 %) are added within 10 min, maintaining the pH value at 4 by the addition of 32.9 ml 30 % NaOH solution. The resulting suspension is stirred for 30 min at a pH value of 4 and then heated up to 30 °C in 25 min. The pH value is adjusted to 9 by the addition of 45.0 ml 30 % NaOH solution. The solution is stirred for 1 hour at 30 °C and a pH value of 9. After filtration the product is precipitated by the addition of 70.5 ml 20 % HCl solution until a pH value of 3.6 is obtained. The product is filtered, washed with 1 I water and then dried for 5 hours at 75 °C and a pressure of 50 mbar on a rotavap. The product is further dried for 15 hours at 75 °C and a pressure oft 125 mbar in an vacuum dryer. 133 g of ferri-succinylcasein are obtained.

### Example 3

120 g casein are suspended in 810 g water at a temperature of 20 °C. The pH value is adjusted to 8 by the addition of 9.3 ml 30 % NaOH solution within 40 min. 20 g succinic anhydride are added in four portions within 20 min. During that time the pH value is maintained at 8 by the addition of 29.4 ml 30 % NaOH solution. The resulting solution is stirred for 30 min, maintaining the pH value at 8 by the addition of 5 ml 30 % NaOH. The succinylcasein is precipitated by adjustment of the pH value to 3.8 with 64.0 ml 20 % HCl solution. 57.2 g of a FeCl₃-solution (12 %) are added within 10 min, maintaining the pH value at 3.8 by the addition of 32.2 ml 30 % NaOH solution. The resulting suspension is stirred for 30 min at a pH value of 3.8 and then heated up to 50 °C in 30 min. The pH value is adjusted to 9 by the addition of 46.8 ml 30 % NaOH solution. The solution is stirred for 1 hour at 50 °C and a pH value of 9. The solution is diluted with water (preheated to 50 °C) in a volume ratio 1 : 4 (reaction solution ; water) and stirred for 5 min at 50 °C. After filtration the product is precipitated by the addition of 74.2 ml 20 % HCl solution until a pH value of 3.6 is obtained, The product is filtered, washed with 1l water and then dried for 24 hours at 75°C and a pressure oft 125 mbar in an vacuum dryer. 130 g of ferri-succinylcasein are obtained.

### Example 4

### (The following example illustrates a preferred embodiment combining concentrated synthesis and partially continuous work-up)

600 g casein are suspended in 4050 g water at a temperature of 20°C. The pH value is adjusted to 8 by the addition of 46 ml 30 % NaOH solution within 40 min. 100 g succinic anhydride are added in four portions within 20 min. During that time the pH value is maintained at 8 by the addition of 124 ml 30 % NaOH solution. The resulting solution is stirred for 30 min, maintaining the pH value at 8. The succinylcasein is precipitated by adjustment of the pH value to 3.8 with 280 ml 20 % HCl solution. 286 g of a FeCl₃-solution (12 %) are added within 10 min, maintaining the pH value at 3.8 by the addition of 170 ml 30 % NaOH solution. The resulting suspension is stirred for 30 min at a pH value of 3.8 and then heated up to 50°C in 25 min. The pH value is adjusted to 9 by the addition of 215 ml 30 % NaOH solution. The solution is stirred for 1 hour at 50°C and a pH value of 9.

For the continuous work-up, one half of the reaction solution is diluted with water of 50°C in a volume ratio 1 : 4 (reaction solution : water) via a static pipe mixer. The liquids are transferred via peristaltic pumps, resulting in a flow of 3.0 - 3.5 l/min for the combined liquids in the static pipe mixer. From the static pipe mixer the diluted solution is directly transferred in a filter device and filtered. The filtrated solution is directly transferred to a second static pipe mixer and precipitated by proportioned addition of 280 ml 20 % HCl, regulating the pH of the product suspension stream to 3.2 - 3.8. The product suspension stream is directly transferred on a filter device and filtered. The product is washed with.2l water and then dried for 48 hours at 75°C and a pressure of 1 25 mbar in a vacuum dryer. 257 g of ferri-succinylcasein are obtained.

### Example 5

100 g casein are suspended in 500 g water at a temperature of 20 °C. The pH value is adjusted to 8 by the addition of 8,3 ml 30 % NaOH solution within 40 min. 16.7 g succinic anhydride are added in four portions within 20 min. During that time the pH value is maintained at 8 by the addition of 25.3 ml 30 % NaOH solution. The resulting solution is stirred for 30 min, maintaining the pH value at 8 by the addition of 1.9 ml 30 % NaOH. The succinylcasein is precipitated by adjustment of the pH value to 3.8 with 49.8 ml 20 % HCl solution. 47.7 g of a FeCl₃-solution (12 %) are added within 10 min, maintaining the pH value at 3.8 by the addition of 28.6 ml 30 % NaOH solution. The resulting suspension is stirred for 30 min at a pH value of 3.8 and then heated up to 30 °C in 30 min. The pH value is adjusted to 9 by the addition of 32.8 ml 30 % NaOH solution. The solution is stirred for 1 hour at 30 °C and a pH value of 9. The solution is diluted with water (of room temperature) in a volume ratio 1 : 6 (reaction solution : water) and stirred for 5 min. After filtration the product is precipitated by the addition of 43.8 ml 20 % HCl solution until a pH value of 3.6 is obtained. The product is filtered, washed with 0.8 l water and then dried for 20 hours at 75 °C and a pressure of 1 25 mbar in an vacuum dryer. 78 g of ferri-succinylcasein are obtained.

The following table shows the analytical results of the materials synthesized in examples 1-5. For the purpose of comparison the analytical data for the product described in EP 0939083 are given.

**Table - Analytical Results (Examples 1 to 5)**

| Analytics | Product according to EP 0939083* | Example 1 * | Example 2 | Example 3 | Example 4 | Example 5 |
|---|---|---|---|---|---|---|
| Total iron content | 5.4 % | 5.2 % | 5.1 % | 5.4 | 5.1 % | 5.2 % |
| Chloride content | 1.6 % | 0.5% | 0.4 % | 1.1 % | 1.0 % | 0.3% |
| Protein content | 76.5% | 78.7 % | 80.0 % | 79.5% | 78.5% | 77.5 |
| Free succinic acid content | 0.9% | 0.2% | 0.6 % | 0.5 | 0.6 % | 0.3% |
| Total succinic acid content | 8.0 % | 8.8% | 8.3% | 7.6 | 9.0 % | 8.0 % |
| pH aq. Suspension | 2.7 | 3.1 | 3.1 | 3.0 | 3.2 | 3.3 |

| | | | | | | |
|---|---|---|---|---|---|---|
| * : Content of methyl parahydroxybenzoate = 1.0 %, propyl parahydroxybenzoate = 0.45%. | | | | | | |

The IR spectra (in KBr) of the materials obtained in examples 1 to 5 support the structure of ferri-succinylcasein.

## Claims

1. A process for the preparation of ferro-succinylcasein comprising the following steps:
(a) reacting casein with at least one succinylation agent to form an aqueous suspension of succinylcasein, and
(b) reacting the aqueous suspension of succinylcasein obtained in step (a) with at least one iron salt to form ferro-succinylcasein.

2. The process according to claim 1, wherein step (a) comprises the steps:
(a1) suspending casein in water,
(a2) if necessary, adjusting the pH value of the aqueous suspension to at least 6,
(a3) adding at least one succinylation agent, while maintaining a pH value of at least 6 by the addition of at least one base.

3. The process according to claims 1 or 2, wherein step (a) further comprises the step:
(a4) after the completion of the addition of the succinylation agent, precipitation of the succinylcasein obtained by adjusting the pH value to about 2 to 7.0, to obtain an aqueous suspension of succinylcasein having a pH value of about 2 to 7.0.

4. The process according to any of claims 1 to 3, wherein step (b) comprises:
(b1) Adding at least one iron salt to an aqueous suspension of succinylcasein.

5. The process according to any of claims 1 to 4, wherein step (b) comprises the step:
(b2) Adding at least one iron salt to an aqueous suspension of succinylcasein while maintaining a pH value of the aqueous suspension of at least 2 by the addition of at least one base, to obtain ferro-succinylcasein.

6. The process according to any of claims 1 to 5, which comprises the step:
(c) Dissolving ferro-succinylcasein obtained by the addition of at least one base, to form an aqueous composition comprising dissolved ferro-succinylcasein.

7. The process according to any of claims 1 to 6, which further comprises the steps:
(d) Separating insoluble matter from the aqueous composition comprising dissolved ferro-succinylcasein to obtain an aqueous solution of ferro-succinylcasein,
(e) Precipitating ferro-succinylcasein from said aqueous solution by adding at least one acid, and recovering the ferrosuccinylcasein obtained, and
(f) Drying said ferro-succinylcasein obtained, or further processing the wet ferro-succinylcasein obtained directly into a pharmaceutical formulation.

8. The process according to any of claims 1 to 7, which comprises the following steps:
(a) Reacting casein with at least one succinylation agent to form an aqueous suspension of succinylcasein,
(b) Reacting said aqueous suspension of succinylcasein obtained in step (a) with at least one iron salt to form ferro-succinylcasein,
(c) Dissolving said ferro-succinylcasein by the addition of at least one base, to form an aqueous composition comprising dissolved ferro-succinylcasein,
(d) Separating insoluble matter from said aqueous composition comprising dissolved ferro-succinylcasein to obtain an aqueous solution of ferro-succinylcasein,
(e) Precipitating ferro-succinylcasein from said aqueous solution by adding at least one acid, and recovering the ferro-succinylcasein obtained, and
(f) Drying said ferro-succinylcasein obtained, or further processing the wet ferro-succinylcasein obtained directly into a pharmaceutical formulation.

9. The process according to any of claims 1 to 8, wherein in step (a) the succinylation agent is added to the casein in at least two temporally separated portions.

10. The process according to any of claims 1 to 9, wherein in step (b) an aqueous solution of at least one iron salt is added.

11. The process according to any of claims 1 to 10, which comprises further the step of manufacturing a pharmaceutical dosage form from the ferro-succinylcasein obtained.

12. The process according to claim 11, wherein the dosage form is for oral administration,

13. The process according to claim 11, wherein the dosage form is a liquid formulation, including drinkable vials, syrups, elixirs, solutions, suspensions, juices.

## Patentansprüche

1. Ein Verfahren zur Herstellung von Ferro-Succinylcasein, das die folgenden Schritte umfasst:
(a) Umsetzen von Casein mit mindestens einem Succinylierungsmittel zur Bildung einer wässrigen Succinylcasein-Suspension, und
(b) Umsetzen der in Schritt (a) gewonnenen wässrigen Succinylcasein-Suspension mit mindestens einem Eisensalz zur Bildung von Ferro-Succinylcasein.

2. Das Verfahren nach Anspruch 1 , wobei Schritt (a) die Schritte umfasst:
(a1) Suspendieren von Casein in Wasser,
(a2) falls nötig, Einstellen des pH-Wertes der wässrigen Suspension auf mindestens 6,
(a3) Hinzufügen mindestens eines Succinylierungsmittels unter Aufrechterhaltung eines pH-Wertes von mindestens 6 durch Hinzufügen mindestens einer Base.

3. Das Verfahren nach den Ansprüchen 1 und 2, wobei Schritt (a) ferner den Schritt umfasst:
(a4) nach Abschluss des Hinzufügens des Succinylierungsmittels, Ausällen des gewonnenen Succinylcaseins durch Einstellen des pH-Wertes auf etwa 2 bis 7,0, um eine wässrige Succinylcasein-Suspension mit einem pH-Wert von etwa 2 bis 7,0 zu gewinnen.

4. Das Verfahren nach einem der Ansprüche 1 bis 3, wobei Schritt (b) umfasst:
(b1) Hinzufügen von mindestens einem Eisensalz zu einer wässrigen Succinylcasein-Suspension.

5. Das Verfahren nach einem der Ansprüche 1 bis 4, wobei Schritt (b) den Schritt umfasst:
(b2) Hinzufügen von mindestens einem Eisensalz zu einer wässrigen Succinylcasein-Suspension unter Aufrechterhaltung eines pH-Wertes der wässrigen Suspension von mindestens 2 durch Hinzufügen mindestens einer Base, um Ferro-Succinylcasein zu gewinnen.

6. Das Verfahren nach einem der Ansprüche 1 bis 5, das den Schritt umfasst:
(c) Auflösen von gewonnenem Ferro-Succinylcasein durch das Hinzufügen mindestens einer Base, um eine wässrige Zusammensetzung zu bilden, die gelöstes Ferro-Succinylcasein umfasst.

7. Das Verfahren nach einem der Ansprüche 1 bis 6, das ferner die Schritte umfasst:
(d) Abtrennen von unlöslichem Material aus der wässrigen Zusammensetzung, die gelöstes Ferro-Succinylcasein umfasst, um eine wässrige Ferro-Succinylcasein-Lösung zu gewinnen,
(e) Ausfällen von Ferro-Succinylcasein aus der wässrigen Lösung durch Hinzufügen mindestens einer Säure, und Isolieren des gewonnenen Ferro-Succinylcaseins, und
(f) Trocknen des gewonnenen Ferro-Succinylcaseins oder direkte Weiterverarbeitung des gewonnenen nassen Ferro-Succinylcaseins zu einer pharmazeutische Zubereitung.

8. Das Verfahren nach einem der Ansprüche 1 bis 7, das die folgenden Schritte umfasst:
(a) Umsetzen von Casein mit mindestens einem Succinylierungsmittel zur Bildung einer wässrigen Succinylcasein-Suspension,
(b) Umsetzen der in Schritt (a) gewonnenen wässrigen Succinylcasein-Suspension mit mindestens einem Eisensalz zur Bildung von Ferro-Succinylcasein,
(c) Lösen des Ferro-Succinylcaseins durch das Hinzufügen mindestens einer Base, um eine wässrige Zusammensetzung zu bilden, die gelöstes Ferro-Succinylcasein umfasst,
(d) Abtrennen von unlöslichem Material aus der wässrigen Zusammensetzung, die gelöstes Ferro-Succinylcasein umfasst, um eine wässrige Ferro-Succinylcasein-Lösung zu gewinnen,
(e) Ausfällen von Ferro-Succinylcasein aus der wässrigen Lösung durch Hinzufügen mindestens einer Säure, und Isolieren des gewonnenen Ferro-Succinylcaseins, und
(g) Trocknen des gewonnenen Ferr-Succinylcaseins oder direkte Weiterverarbeitung des gewonnenen nassen Ferro-Succinylcaseins zu einer pharmazeutische Zubereitung.

9. Das Verfahren nach einem der Ansprüche 1 bis 8, wobei in Schritt (a) das Succinylierungsmittel dem Casein in mindestens zwei zeitlich getrennten Portionen hinzugefügt wird.

10. Das Verfahren nach einem der Ansprüche 1 bis 9, wobei in Schritt (b) eine wässrige Lösung mindestens eines Eisensalzes hinzugefügt wird.

11. Verfahren nach einem der Ansprüche 1 bis 10, welches einen weiteren Schritt der Herstellung einer pharmazeutischen Dosierungsform aus dem erhaltenen Ferrosuccinylcasein umfasst,

12. Das Verfahren nach Anspruch 1 1 , wobei die Dosierungsform zur oralen Verabreichung bestimmt ist.

13. Das Verfahren nach Anspruch 1 1 , wobei die Dosierungsform eine flüssige Zubereitung ist, einschließlich von trinkbaren Phiolen, Sirupen, Elixieren, Lösungen, Suspensionen und Säften.

## Revendications

1. Procédé pour la préparation de ferro-succinylcaséine, comprenant les étapes suivantes consistant à :
(a) faire réagir de la caséine avec au moins un agent de succinylation pour obtenir une suspension aqueuse de succinylcaséine ; et
(b) faire réagir la suspension aqueuse de succinylcaséine obtenue à l'étape (a) avec au moins un sel de fer pour obtenir de la ferro-succinylcaséine.

2. Procédé selon la revendication 1, dans lequel l'étape (a) comprend les étapes consistant à :
(a1) mettre de la caséine en suspension dans de l'eau ;
(a2) si nécessaire, régler la valeur de pH de la suspension aqueuse à au moins 6 ;
(a3) ajouter au moins un agent de succinylation, tout en maintenant une valeur de pH d'au moins 6 par l'addition d'au moins une base.

3. Procédé selon la revendication 1 ou 2, dans lequel l'étape (a) comprend en outre l'étape consistant à :
(a4) après l'achèvement de l'addition de l'agent de succinylation, soumettre la succinylcaséine obtenue à une succinylation en réglant la valeur de pH à une valeur d'environ 2 à 7,0, afin d'obtenir une suspension aqueuse de succinylcaséine possédant une valeur de pH d'environ 2 à 7,0.

4. Procédé selon l'une quelconque des revendications 1 à 3, dans lequel l'étape (b) comprend le fait de :
(b1) ajouter au moins un sel de fer à une suspension aqueuse de succinylcaséine.

5. Procédé selon l'une quelconque des revendications 1 à 4, dans lequel l'étape (b) comprend l'étape consistant à :
(b2) ajouter au moins un sel de fer à une suspension aqueuse de succinylcaséine tout en maintenant une valeur de pH de la suspension aqueuse à au moins 2 par l'addition d'au moins une base, pour obtenir de la ferro-succinylcaséine.

6. Procédé selon l'une quelconque des revendications 1 à 5, qui comprend l'étape consistant à :
(c) dissoudre de la ferro-succinylcaséine obtenue par l'addition d'au moins une base, pour obtenir une composition aqueuse comprenant de la ferro-succinylcaséine dissoute.

7. Procédé selon l'une quelconque des revendications 1 à 6, qui comprend en outre les étapes consistant à :
(d) séparer la matière insoluble de la composition aqueuse comprenant de la ferro-succinylcaséine dissoute pour obtenir une solution aqueuse de ferro-succinylcaséine ;
(e) précipiter la ferro-succinylcaséine de ladite solution aqueuse par addition d'au moins un acide et récupérer la ferro-succinylcaséine obtenue ; et
(f) sécher ladite ferro-succinylcaséine obtenue ou bien soumettre à un traitement supplémentaire là ferro-succinylcaséine humide obtenue pour la transformer directement en une formulation pharmaceutique.

8. Procédé selon l'une quelconque des revendications 1 à 7, qui comprend les étapes suivantes consistant à :
(a) faire réagir de la caséine avec au moins un agent de succinylation pour obtenir une suspension aqueuse de succinylcaséine :
(b) faire réagir ladite suspension aqueuse de succinylcaséine obtenue à l'étape (a) avec au moins un sel de fer pour obtenir de la ferro-succinylcaséine ;
(c) dissoudre ladite ferro-succinylcaséine par l'addition d'au moins une base, pour obtenir une composition aqueuse comprenant de la ferro-succinylcaséine dissoute ;
(d) séparer la matière insoluble de ladite composition aqueuse comprenant de la ferro-succinylcaséine dissoute, afin d'obtenir une solution aqueuse de ferro-succinylcaséine ;
(e) précipiter la ferro-succinylcaséine de ladite solution aqueuse par addition d'au moins un acide et récupérer la ferro-succinylcaséine obtenue ; et
(f) sécher ladite ferro-succinylcaséine obtenue ou bien soumettre à un traitement supplémentaire la ferro-succinylcaséine humide obtenue pour la transformer directement en une formulation pharmaceutique.

9. Procédé selon l'une quelconque des revendications 1 à 8, dans lequel, à l'étape (a), l'agent de succinylation est ajouté à la caséine en au moins deux portions séparées dans le temps.

10. Procédé selon l'une quelconque des revendications 1 à 9, dans lequel, à l'étape (b), on ajoute une solution aqueuse d'au moins un sel de fer.

11. Procédé selon l'une quelconque des revendications 1 à 10, qui comprend en outre l'étape consistant à préparer une forme posologique pharmaceutique à partir de la ferro-succinylcaséine obtenue.

12. Procédé selon la revendication 11, dans lequel la forme posologique est destinée à une administration par voie orale.

13. Procédé selon la revendication 11, dans lequel la forme posologique est une formulation liquide, y compris des fioles buvables, des sirops, des élixirs, des solutions, des suspensions, des jus.
